(19) Europäisches Patentamt
European Patent Office
Office européen des brevets

(11) Publication number: **0 272 115 B1**

(12) **EUROPEAN PATENT SPECIFICATION**

(45) Date of publication of patent specification: **13.10.93**

(51) Int. Cl.5: **C09K 19/12**, C07C 43/225, C07C 43/205, C07C 255/52

(21) Application number: **87311104.1**

(22) Date of filing: **16.12.87**

(54) **Optically active liquid crystalline biphenyl compound.**

(30) Priority: **16.12.86 JP 299615/86**

(43) Date of publication of application:
**22.06.88 Bulletin 88/25**

(45) Publication of the grant of the patent:
**13.10.93 Bulletin 93/41**

(84) Designated Contracting States:
**CH DE FR GB LI**

(56) References cited:
**EP-A- 0 131 373**
**EP-A- 0 269 062**
**US-A- 4 257 911**

**ACTA CHEMICA SCANDINAVICA, vol. B 31, no. 9, 1977, pages 813-817; L. ERBERSON et al.: "Studies on electrolytic substitution reactions. XII. Synthesis of 4-alkoxy-4'-cyanobiphenyls - a class of liquid crystals - via anodic cyanation of 4,4'-dialkoxybiphenyls in emulsion systems"**

(73) Proprietor: **Chisso Corporation**
**6-32, Nakanoshima 3-chome**
**Kita-ku**
**Osaka-shi Osaka-fu(JP)**

(72) Inventor: **Miyazawa, Kazutoshi**
**12-14, Mutsuura 2-chome**
**Kanazawa-ku**
**Yokohama-shi Kanagawa-ken(JP)**
Inventor: **Inukai, Takashi**
**4-46, Mori 3-chome**
**Isogo-ku**
**Yokohama-shi Kanagawa-ken(JP)**
Inventor: **Inoue, Hiromichi**
**10-2, Otsutomo-cho**
**Kanazawa-ku**
**Yokohama-shi Kanagawa-ken(JP)**
Inventor: **Saito, Shinichi**
**10-1, Otsutomo-cho**
**Kanazawa-ku**
**Yokohama-shi Kanagawa-ken(JP)**
Inventor: **Ohno, Kouji**
**10-3, Otsutomo-cho**
**Kanazawa-ku**
**Yokohama-shi Kanagawa-ken(JP)**

(74) Representative: **Lamb, John Baxter et al**
**MARKS & CLERK**
**57-60 Lincoln's Inn Fields**
**London WC2A 3LS (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.6/3.3.1)

## Description

BACKGROUND OF THE INVENTION

1. Field of the Invention

This invention relates to a novel organic compound and a liquid crystal composition containing the same. More particularly it relates to a biphenyl compound useful as a component of a chiral smectic liquid crystal composition useful for light switching elements making use of ferroelectric liquid crystals.

2. Description of the Related Art

At present, TN (Twisted Nematic) mode display has been most broadly employed, but it is inferior in the aspect of response rate to emissive mode display elements (electroluminescence, plasma display, etc.). Thus, various attempts directed to improvement in this respect has been made, but it does not appear that a possibility of improvement therein to a large extent remains. Thus, various liquid display devices based on another principle has been attempted, and among these, there is a display mode utilizing ferroelectric liquid crystals (N.A. Clark et at, Applied Phys. lett., 36, 899 (1980)). This mode utilizes chiral smectic C phase (hereinafter abbreviated to SC* phase) or chiral smectic H phase (hereinafter abbreviated to SH* phase) of ferroelectric liquid crystals.

As one of specific features required for chiral smectic liquid crystals utilized for the display mode utilizing ferroelectric liquid crystals, the large spontaneous polarization value Ps thereof is mentioned, but among so far known chiral smectic liquid crystals, those known to have a sufficiently large Ps value have been few. Liquid crystalline biphenyl compounds containing optically active substituents are disclosed in EP-A-0131373 and EP-A-0269062.

The present inventors have searched for a compound which, when used as a component of chiral smectic liquid crystal compositions, increases mainly their Ps value, and as a result have attained the present invention.

The present invention resides in:

a biphenyl compound expressed by the formula

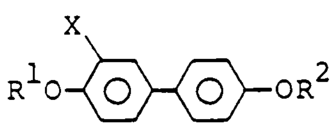

(I)

wherein X represents fluorine atom, chlorine atom, bromine atom, cyano group or hydrogen atom; when X represents fluorine atom, chlorine atom, bromine atom or cyano group, either one of $R^1$ or $R^2$ represents an alkyl group of 4 to 18 carbon atoms having a methyl branch and the other represents an alkyl Group of 1 to 20 carbon atoms; and when X represents hydrogen atom, either one of $R^1$ or $R^2$ represents an alkyl group having a methyl branch expressed by the formula

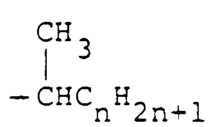

wherein n represents an integer of 3 or more, and the other represents an alkyl group of 1 to 20 carbon atoms; excluding the compound in which $R^1$ is a 6-methyl-octyl group, $R^2$ is an n-decyl group and X is a fluorine atom. The invention also provides a chiral smectic or chiral nematic composition containing at least one kind of biphenyl compounds expressed by formula (I).

The compound of the formula (I) includes an optically active substance based on the methyl group and a racemic substance thereof, and both the substances have a common advantage as a component of chiral smectic compositions. Further, as illustrated below, the compound includes those which exhibit smectic liquid crystal phase by themselves and those which do not exhibit the phase, but the both have the same effectiveness as a component of chiral smectic compositions.

The phase transition points and spontaneous polarization values of the representatives of the compound of the formula (I) are shown in Table 1.

Table 1

| Compound No. | In formula (I) $R^1$ | In formula (I) $R^2$ | In formula (I) X | Optical activity | C | | SX *1) | SC* | I | Remark |
|---|---|---|---|---|---|---|---|---|---|---|
| 1 | $-\underset{*}{\overset{CH_3}{\overset{\vert}{C}}}HC_6H_{12}$ | $-C_6H_{13}$ | F | S | · | 33.3 | — | — | · | Example 1 |
| 2 | $-C_8H_{17}$ | $-\underset{*}{\overset{CH_3}{\overset{\vert}{C}}}HC_6H_{13}$ | F | S | · | 31.0 | — | — | · | |
| 3 | $-C_{10}H_{21}$ | $-\underset{*}{\overset{CH_3}{\overset{\vert}{C}}}HC_6H_{13}$ | H | R | · | 62.4 | — | — | · | |
| 4 | $-\underset{*}{\overset{CH_3}{\overset{\vert}{C}}}HC_6H_{13}$ | $-C_{10}H_{21}$ | CN | S | · | 0.0 | — | — | · | Example 2 |

*1): SX phase represents an unidentified smectic phase.

*2): The value shows an extrapolation one at 25°C sought from a mixture of the compound in 20% with another liquid crystal having SC phase.

Most of the compounds (I) of the present invention exhibit no ferroelectric liquid crystal phase by themselves, but when they are added to another material having chiral smectic C phase (SC* phase) or another material having smectic C phase (SC phase), a liquid crystal material having an extremely large spontaneous polarization value is obtained. The spontaneous polarization value of the compounds of the formula (I) presumed according to extrapolation method amounts to about 94 $nC/cm^2$. It is seen that this value is extremely large as compared with about 1 $nC/cm^2$ as measured by the present inventors, of the

spontaneous polarization value of a chiral smectic compound expressed by the formula

(+) 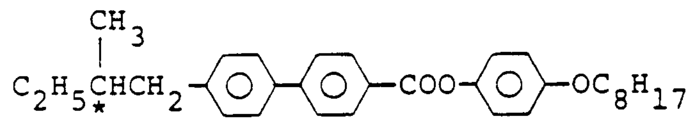

which is disclosed in Japanese patent application laid-open No. Sho 53-22883/1978.

Further, one more superior specific feature of the biphenyl compounds of the formula (I) of the present invention consists in a low viscosity.

As described above, a superior advantage of the display elements utilizing chiral smectic liquid crystal composition consists in that the response rate is low, that is, the time during which molecules are inverted by an electric field is short, and since the inversion is not inhibited due to the low viscosity, it can be said that the biphenyl compounds of the present invention have a superior specific feature as a component of chiral smectic liquid crystal compositions.

Now, preferred examples of materials having smectic phase and used in admixture with the compounds of the formula (I) are 5-alkyl-2-(4-alkoxyphenyl)pyrimidines represented by

5-octyl-2-(4-octyloxyphenyl)pyrimidine,
5-octyl-2-(4-nonyloxyphenyl)pyrimidine,
5-octyl-2-(4-decyloxyphenyl)pyrimidine, etc.;

5-alkyl-2-(4-alkoxyphenyl)pyridines represented by

5-heptyl-2-(4-octyloxyphenyl)pyridine,
5-octyl-2-(4-octyloxyphenyl)pyridine,
5-heptyl-2-(4-nonyloxyphenyl)pyridine,
5-nonyl-2-(4-nonyloxyphenyl)pyridine, etc.;

4-alkoxyphenyl 4-alkoxybenzoates represented by

4-octyloxyphenyl 4-octyloxybenzoate,
4-nonyloxyphenyl 4-octyloxybenzoate,
4-decyloxyphenyl 4-nonyloxybenzoate, etc.; etc.

Further, preferred examples of materials having chiral smectic phase and used in admixture with the compounds of the formula (I) are optically active pyrimidine compounds represented by

5-octyl-2-[4-(6-methyloctyloxy)phenyl]pyrimidine,
5-octyl-2-[3-fluoro-4-(8-methyldecyloxy)phenylpyrimidine, etc.;

optically active pyridine compounds represented by

5-octyl-2-[4-(4-methylhexyloxy)phenyl]pyridine,
5-octyl-2-[3-fluoro-4-(6-methyloctyloxy)phenyl]pyridine, etc.;

optically active ester compounds represented by

4-octyloxyphenyl 4-(1-methylheptyloxy)biphenyl-4'-carboxylate,
4-hexylphenyl 3-fluoro-4-(1-methylheptyloxy)biphenyl-4'-carboxylate, etc.; etc.

Further, among the compounds of the formula (I), optically active substances have an optically active carbon atom; hence when they are added to nematic liquid crystals, they have a capability of inducing a twisted structure. Nematic liquid crystals having a twisted structure i.e. chiral nematic liquid crystals do not form the so-called reverse domain dechiralization lines of TN type display elements; hence the compounds of the formula (I) can be used as an agent for preventing the reverse domain from forming.

Further, the temperature change in the pitch of the chiral nematic liquid crystals exhibits a behavior directly opposite to that of various currently known compounds, that is, a behavior that the intrinsic pitch induced when the compounds of the present invention are added to nematic liquid crystal materials, shrinks with temperature rise.

The temperature change in the pitch P of the chiral nematic liquid crystals is expressed by the formula

$$\delta P = \frac{2\ (\ P(t_1)\ -\ P(t_2)\ )}{P(t_1)\ +\ P(t_2)} \times \frac{100}{t_1 - t_2}$$

wherein t represents a temperature and P(t) represents a pitch at t°C. When the temperature change in a chiral pitch as measured at 20°C and 60°C, of a mixture obtained by adding (S)-4-(2-methylbutyl)-4'-cyanobiphenyl as a known often used chiralizing agent in 1% to a commercially available nematic liquid crystal composition (ZLI-1132, trademark of a product manufactured by Merck Company), the resulting δP is 0.543. Whereas the δP of a mixture obtained by adding a compound of the present invention (compound No. 4 in Table 1) in 1% to the ZLI-1132 is -0.641, that is, a reverse temperature change is exhibited. Thus, when the compound of the present invention in a suitable quantity to another chiralizing substance, it is possible to obtain a chiral nematic liquid crystal composition having a small temperature change in the chiral pitch P.

Preparation of the compound of the present invention:

Compounds of the formula (I) wherein X represents F or Cℓ may be suitably prepared through the following passageway:

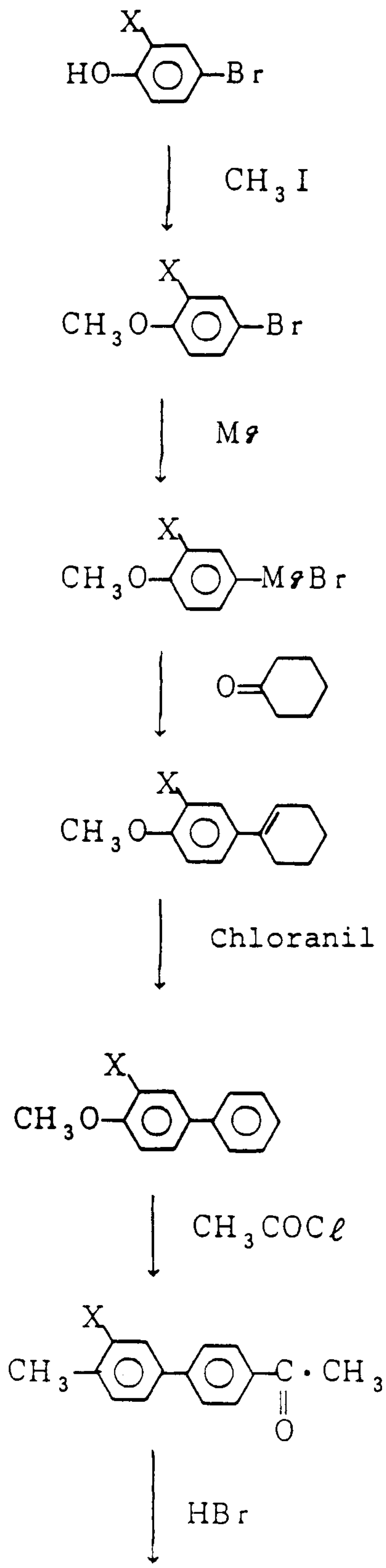


(1)

(2)

(3)

(4)

(5)

(6)

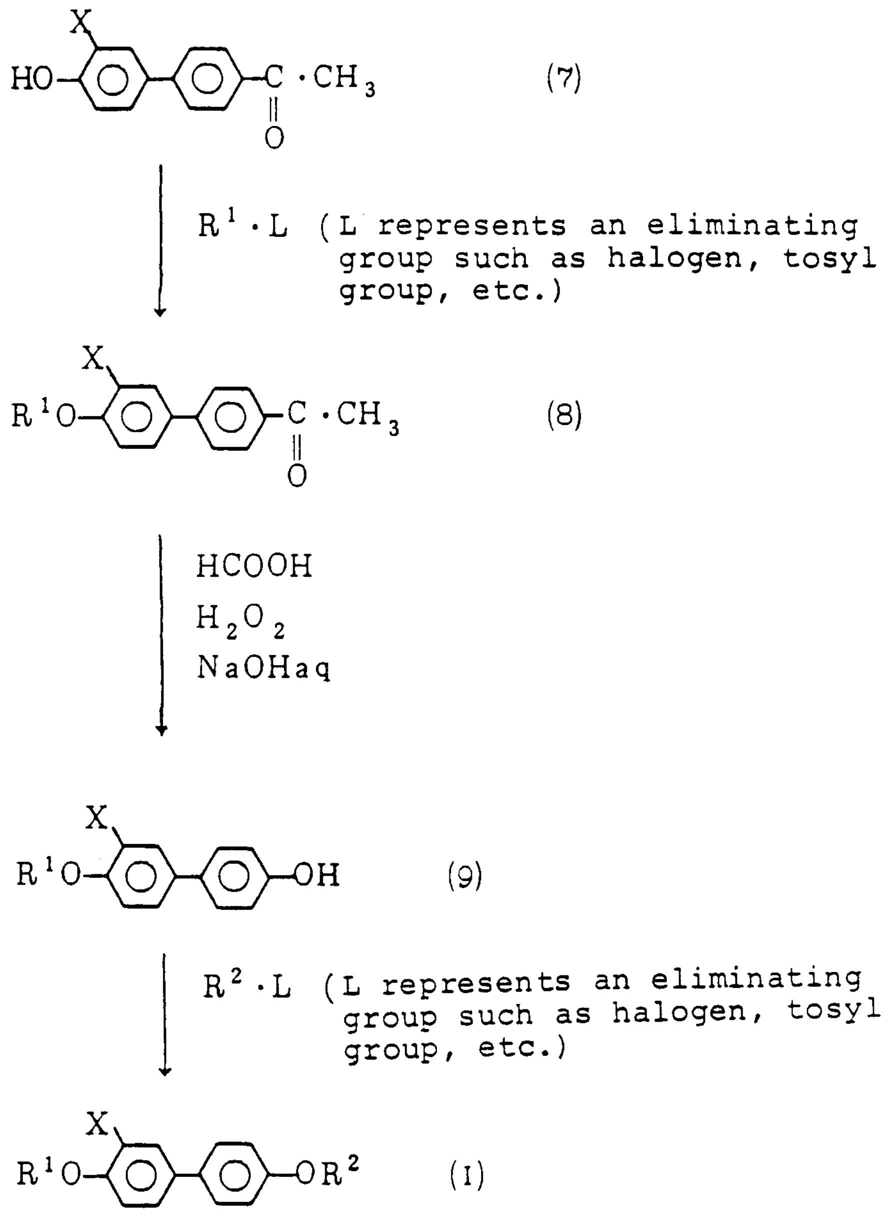


Wherein $R^1$, $R^2$, and X are as defined above.

Namely, a known 2-halogeno-4-bromophenol (1) is methyl-etherified to obtain a compound (2), which is reacted with metal magnesium to obtain a Grignard reagent (3), which is reacted with cyclohexanone to obtain a compound (4), which is reacted with a dehydrogenating agent such as chloranil to obtain a compound (5), which is reacted with acetyl chloride to obtain a compound (6), which is demethylated to obtain a compound (7), which is reacted with an alkylhalide or an alkyl tosylate to obtain a compound (8), which is reacted with performic acid and then with an alkali to obtain a compound (9), which is reacted with an alkyl halide or an alkyl tosylate to obtain the objective compound (1).

Further, among the compounds of the formula (I), those wherein X represents CN or Br may be suitably prepared through the following passageways:

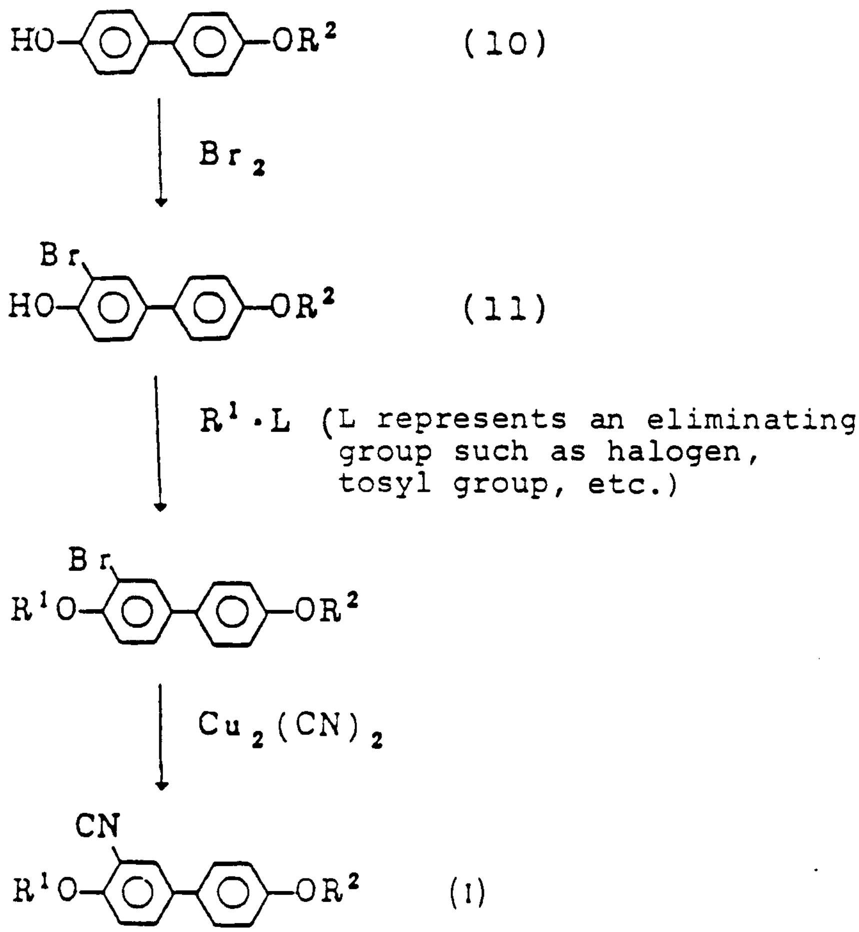


wherein $R^1$ and $R^2$ are as defined above.

Namely, bromine is reacted with a 4-alkyloxy-4'-phenylphenol (10) to obtain a compound (11), which is then reacted with an alkyl halide or an alkyl tosylate, followed by subjecting the resulting compound to etherification according to a known method to obtain a compound of the formula (I) wherein X represents Br.

Further, compounds of the formula (I) wherein X represents CN or Br may be suitably prepared through the following passageways:

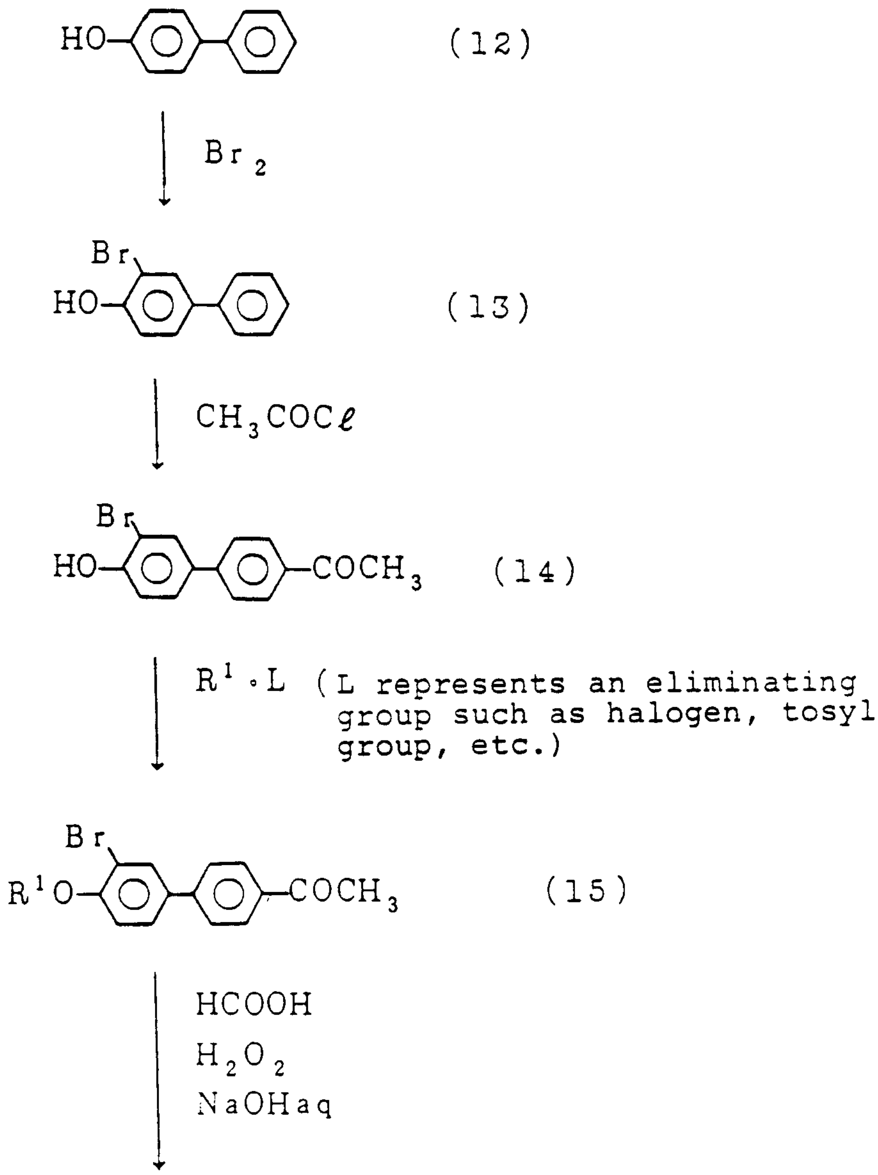
(12)
$Br_2$
(13)
$CH_3COC\ell$
(14)
$R^1 \cdot L$ (L represents an eliminating group such as halogen, tosyl group, etc.)
(15)
HCOOH
$H_2O_2$
NaOHaq

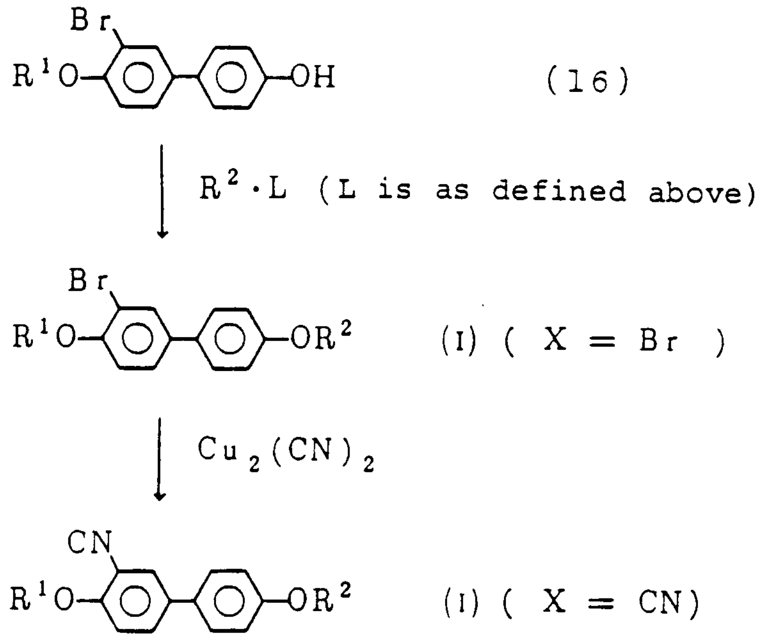


(16)

(I) ( X = Br )

(I) ( X = CN)

Namely, bromine is reacted with p-phenylphenol (12) to obtain a compound (13), which is then reacted with acetyl chloride in the presence of a catalyst to obtain a compound (14), which is reacted with an alkyl halide or an alkyl tosylate to obtain a compound (15), which is reacted with performic acid and then with an alkali to obtain a compound (16), which is reacted with an alkyl halide or an alkyl tosylate to obtain a compound of the formula (I) wherein X = Br. This compound is cyanogenated with a cyanogenating agent such as copper (1) cyanide to obtain a compound of the formula (I) wherein X = CN.

Further, a compound of the formula (I) wherein X represents H may be suitably prepared through the following passageways:

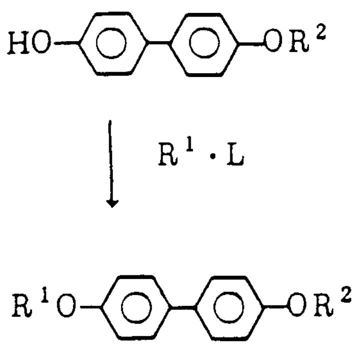


(17)

(I)

wherein $R^1$ and $R^2$ are each as defined above.

Namely, an alkyl halide or an alkyl tosylate is reacted with a 4-alkyloxy-4′-phenylphenol (17) to carry out etherification according to a known method to obtain the objective compound of the formula (I).

In addition, in any of the above-mentioned cases, when either one of $R^1 \cdot L$ or $R^2 \cdot L$ (wherein L represents an eliminating group such as halogen or tosyl group) as a raw material for introducing $R^1$ or $R^2$ is an optically active substance, the resulting compound of the formula (I) is also an optically active substance, while in the case where a racemic substance is used as the raw material, the finally objective substance is also a racemic substance.

The compound and liquid crystal composition of the present invention will be described in more detail by way of Examples.

Example 1

Preparation of optically active 3-fluoro-4-(1-methylheptyloxy)-4'-hexyloxybiphenyl (a compound of the formula (I) wherein

$R^1$ = 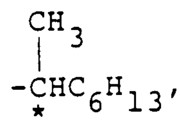,

$R^2$ = $-C_6H_{13}$ and X = F; compound No. 1)

2-Fluoro-4-bromophenol (382.0 g, 2.0 mols) was dissolved in ethanol (1,000 mℓ), followed by adding KOH (113.0 g, 2.0 mols) to the solution, sufficiently agitating the mixture, dropwise adding methyl iodide (312.3 g, 2.2 mols), refluxing the mixture for 4 hours, distilling off most portion of ethanol, distilling the residue under reduced pressure and collecting a fraction at 65° to 66°C (4.5 mmHg) to obtain 2-fluoro-4-bromoanisole (328.0 g), which was then reacted with magnesium (38.9 g) in diethyl ether to obtain 2-fluoro-4-anisylmagnesium bromide, followed by dropwise adding cyclohexanone (157.0 g, 1.6 mol) at 7°C or lower, further agitating the mixture at room temperature for about 30 minutes, adding 6N-HCℓ (350 mℓ), refluxing the mixture for one hour, washing the resulting organic layer with water, distilling off diethyl ether and recrystallizing the residue from ethanol to obtain 3-fluoro-4-anisylcyclohexene-1 (180.4 g) having a m.p. of 49.0° to 50.0°C. This 3-fluoro-4-anisylcyclohexene-1 (112.0 g, 0.55 mol) was dissolved in xylene (700 mℓ), followed by adding p-chloranil (267.0 g, 1.1 mol) to the solution, refluxing the mixture for 4 hours, filtering off the resulting tetrachlorohydroquinone, washing the filtrate with 2N-NaOH aqueous solution, distilling off the solvent, subjecting the residue to column chromatography using a column filled with activated alumina and recrystallizing from ethanol to obtain 3-fluoro-4-methoxybiphenyl (m.p. 84.5° to 86.0°C, 69.2 g).

This 3-fluoro-4-methoxybiphenyl (50.0 g, 0.25 mol) was dissolved in carbon disulfide (260 mℓ), followed by adding anhydrous aluminum chloride (39.5 g, 0.296 mol), dropwise adding a solution of acetyl chloride (21.4 g, 0.27 mol) dissolved in carbon disulfide (80 mℓ) at 10°C or lower, refluxing the mixture for 3 hours, feeding the resulting material into 6N-HCℓ (1 ℓ), collecting the resulting deposited crystals and recrystallizing from toluene to obtain 3-fluoro-4-methoxy-4'-acetylbiphenyl (49.4 g) having a m.p. of 144.7°C - 145.7°C.

To this 3-fluoro-4-methoxy-4'-acetylbiphenyl (40.0 g, 0.17 mol) were added acetic acid (450 mℓ) and hydrobromic acid (180.0 g), followed by refluxing the mixture for 35 hours, feeding the reaction material into ice water, and collecting the deposited crystals to obtain 3-fluoro 4-hydroxy-4'-acetylbiphenyl (32.5 g) having a m.p. of 154.7° - 158.5°C.

This 3-fluoro-4-hydroxy-4'-acetylbiphenyl (15.0 g, 0.066 mol) was dissolved in ethanol (370 mℓ), followed by adding KOH (4.0 g, 0.072 mol) and optically active p-toluenesulfonic acid 1-methylheptyl ester (20.5 g, 0.072 mol) to the solution, refluxing the mixture for 5 hours, distilling off most portion of ethanol, adding toluene and water, agitating the mixture, washing the resulting toluene layer with an acid, then with an alkali and further with water, distilling off the solvent and recrystallizing the residue from a mixed solvent of ethanol and ethyl acetate to obtain 3-fluoro-4-(1-methylheptyloxy)-4'-acetylbiphenyl (6.4 g) having a m.p. of 67.6° - 68.5°C.

This 3-fluoro-4-(1-methylheptyloxy)-4'-acetylbiphenyl (6.37 g, 0.019 mol) was dissolved in formic acid (300 g), followed by dropwise adding 30% aqueous hydrogen peroxide (18.1 g, 0.19 mol) to the solution at 50°C, agitating the mixture for 5 hours, as it was, adding 50% aqueous solution of sodium hydrogen sulfite (75 mℓ) at 10°C or lower and further toluene, washing the resulting organic layer with water, distilling off toluene to obtain an oily residue (6.4 g), dissolving this residue in ethanol (45 mℓ), adding a solution of NaOH (7.6 g, 0.19 mol) in water (65 mℓ), refluxing the mixture, feeding the resulting material into 6N-HCℓ aqueous solution and collecting deposited crystals to obtain 3-fluoro-4-(1-methylheptyloxy)-4'-hydroxybiphenyl (5.3 g) having a m.p. of 65.2° - 66.7°C. This product (5.0 g, 0.0165 mol) was dissolved in ethanol (100 mℓ), followed by adding $KCO_3$ (2.5 g, 0.018 mol) and then n-hexylbromide (5.45 g, 0.033 mol) to the solution, refluxing the mixture for 8 hours, distilling off ethanol, adding toluene to the residue, washing the resulting organic layer with water, distilling off toluene and recrystallizing the residue from ethanol to obtain 3-fluoro-4-(1-methylheptyloxy)-4'-hexyloxybiphenyl (4.5 g) having a m.p. of 33.0°C.

Example 2

Preparation of optically active 3-cyano-4-(1-methylheptyloxy)-4'-decyloxybiphenyl (a compound of the formula (I) wherein

$R^1$ = 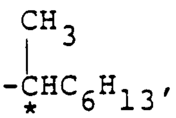

$R^2$ = $-C_{10}H_{21}$ and X = CN; compound No. 7)

4-Hydroxy-4'-decyloxybiphenyl (120.0 g, 0.37 mol) was dissolved in p-dioxane (3 ℓ), followed by gradually dropwise adding bromine (76.4 g, 0.48 mol) to the solution at 15 °C, raising the temperature up to 70 °C, distilling off the solvent, under reduced pressure and recrystallizing the residue from chloroform (1 ℓ) to obtain 3-bromo-4-hydroxy-4'-decyloxybiphenyl (141.0 g) having a m.p. of 77.8 ° - 78.2 °C.

This 3-bromo-4-hydroxy-4'-decyloxybiphenyl (120.0 g, 0.296 mol) was dissolved in ethanol (2 ℓ), followed by adding KOH (16.6 g, 0.296 mol) and then p-toluenesulfonic acid 1-methylheptyl ester (92.0 g, 0.32 mol) to the solution, refluxing the mixture for 8 hours, distilling off ethanol, dissolving the residue in toluene, washing the solution with 2N-NaOH aqueous solution and then with water, distilling off toluene and recrystallizing the residue from a mixed solvent of ethanol and ethyl acetate to obtain 3-bromo-4-(1-methylheptyloxy)-4'-decyloxybiphenyl (41.3 g). This product was oily at room temperature. This product (8.2 g, 0.0158 mol) was dissolved in N-methyl-2-pyrrolidone (40 mℓ), followed by adding copper (1) cyanide (2.84 g, 0.0158 mol) to the solution, agitating the mixture at 190 °C for 6 hours, adding ferric chloride (6.2 g), hydrochloric acid (1.5 mℓ) and water (10 mℓ), agitating the mixture at 95 °C for 2 hours, allowing the mixture to cool down, adding chloroform for extraction, washing the resulting organic layer with water, distilling off the solvent and recrystallizing the residue from aqueous ethanol to obtain 3-cyano-4-(1-methylheptyloxy)-4'-decyloxybiphenyl (6.1 g). This product was oily at room temperature, but its melting point was 0.0 °C according to DSC measurement.

Example 3 (Use example 1)

Using the liquid crystal compounds in the above Table 1 and another liquid crystal compound, the following chiral smectic liquid crystal composition was prepared:

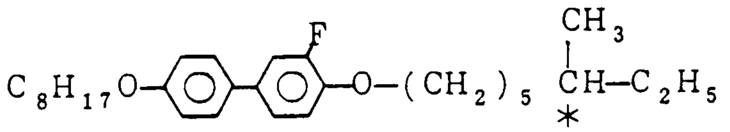 25 wt.%

(Compound No. 2)

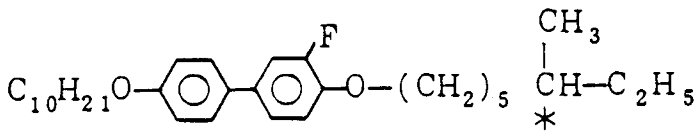 30 wt.%

(Compound No. 3)

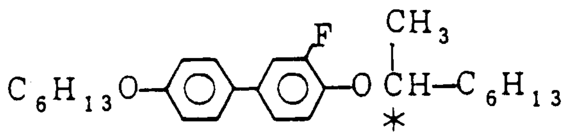 5 wt.%

(Compound No. 1)

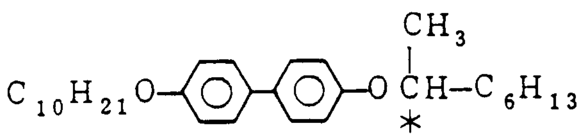 7.5 wt.%

(Compound No. 6)

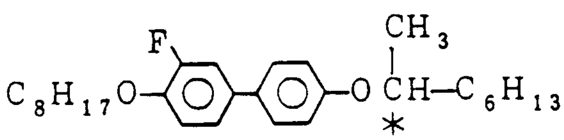 2.5 wt.%

(Compound No. 4)

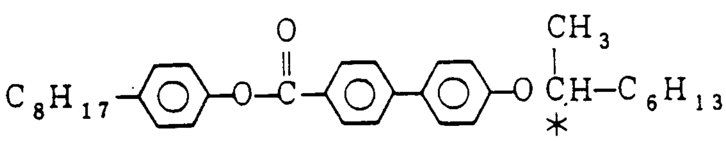 30 wt.%

The above composition was filled in a cell of 2 µm thick provided with transparent electrodes each obtained by applying PVA (polyvinyl alcohol) as an aligning agent and subjecting the resulting surface to parallel aligning treatment, followed by providing the resulting liquid crystal element between two sheets of crossed polarizers and impressing a voltage of 15 V onto the cell. As a result, change in the intensity of transmitted light was observed.

The response time was sought from the change in the intensity of transmitted light to give about 110 µsec at 25 °C.

The above liquid crystal composition had a m.p. of -5 °C, exhibited SI* phase above the temperature, SC* phase at 18 °C and SA phase at 58 °C and formed an isotropic liquid at 65 °C. Its supercooled state was confirmed down to -20 °C, SI* phase was exhibited down to this temperature and other smectic phases were not observed.

In addition, its spontaneous polarization value was 31 nC/cm$^2$ and its tilt angle was 22 °.

Example 4 (Use example 2)

A nematic liquid crystal composition consisting of

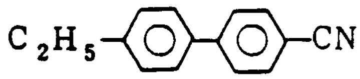 20 wt.%

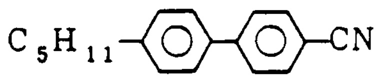 40 wt.%

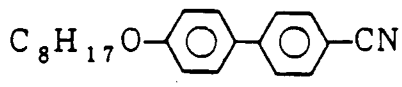 25 wt.%

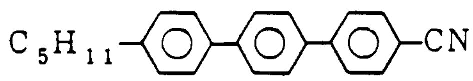 15 wt.%

was filled in a cell provided with transparent electrodes each obtained by applying polyvinyl alcohol (PVA) as an aligning agent and rubbing the resulting surface to subject it to parallel aligning treatment and having a gap between the electrodes of 10 µm to prepare a TN type display cell which was then observed under a polarizing microscope. As a result, a reverse twist domain was observed to be formed.

To the above nematic liquid crystal composition was added a compound of the present invention,

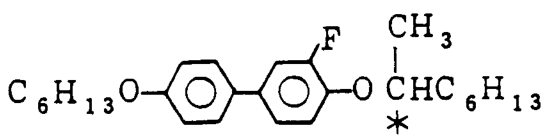 Compound No.1

in 0.1% by weight, and a TN type cell was prepared as above which was similarly observed. As a result, the reverse domain was dissolved and a uniform nematic phase was observed.

## Claims

**1.** Biphenyl compounds of the formula:

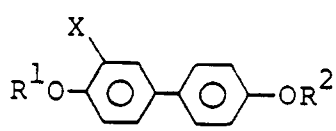 (I)

in which X represents a hydrogen, fluorine, chlorine or bromine atom or a cyano group; and, when X is a fluorine, chlorine or bromine atom or a cyano group, one of $R^1$ and $R^2$ represents a $C_4$-$C_{18}$ alkyl group having a single methyl branch and the other of $R^1$ and $R^2$ represents a $C_1$-$C_{20}$ alkyl group, or, when X represents a hydrogen atom, one of $R^1$ and $R^2$ represents an alkyl group having a methyl branch of the formula

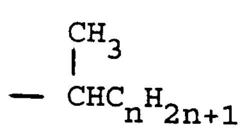

(wherein n represents an integer of 3 or more) and the other of $R^1$ and $R^2$ represents a $C_1$-$C_{20}$ alkyl group; excluding the compound in which $R^1$ is a 6-methyl-octyl group, $R^2$ is an n-decyl group and X is

a flourine atom.

2. A biphenyl compound according to claim 1 characterised in that it is an optically active substance based on the alkyl group having a methyl branch.

3. A biphenyl compound according to claim 1 characterised in that it is in racemic form.

4. A biphenyl compound according to any one of the preceding claims characterised in that X is a hydrogen or fluorine atom or a cyano group.

5. A biphenyl compound according to any one of the preceding claims characterised in that the alkyl group having a methyl branch contains 8 or 9 carbon atoms.

6. A biphenyl compound according to any one of the preceding claims characterised in that the alkyl group other than the alkyl group having a methyl branch is a linear $C_6$-$C_{10}$ alkyl group.

7. A liquid crystal composition comprising at least two components at least one of which is a biphenyl compound as claimed in any one of the preceding claims.

8. A liquid crystal composition according to claim 7, characterised in that it exhibits a chiral smetic phase.

9. A liquid crystal composition according to claim 7, characterised in that it exhibts a chiral nematic phase.

**Patentansprüche**

1. Biphenylverbindungen der Formel:

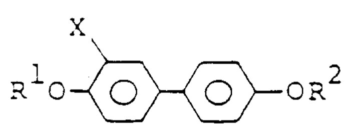

(I)

in der X ein Wasserstoff-, Fluor-, Chlor- oder Bromatom oder eine Cyanogruppe repräsentiert; und, falls X ein Fluor-, Chlor- oder Bromatom oder eine Cyanogruppe repräsentiert, repräsentiert einer der Reste $R^1$ und $R^2$ eine $C_4$-$C_{18}$-Alkylgruppe mit einem einzigen Methylzweig und der andere der Reste $R^1$ und $R^2$ eine $C_1$-$C_{20}$-Alkylgruppe, oder, falls X ein Wasserstoffatom repräsentiert, repräsentiert einer der Reste $R^1$ und $R^2$ eine Alkylgruppe mit einem Methylzweig der Formel

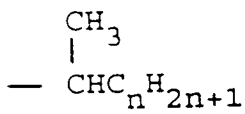

(worin <u>n</u> eine ganze Zahl von 3 oder mehr repräsentiert) und der andere Rest von $R^1$ und $R^2$ repräsentiert eine $C_1$-$C_{20}$-Alkylgruppe; ausgenommen die Verbindung in der $R^1$ eine 6-Methyl-octylgruppe, $R^2$ eine n-Decylgruppe und X ein Fluoratom ist.

2. Biphenylverbindung nach Anspruch 1, dadurch gekennzeichnet, daß sie, basierend auf der Alkylgruppe mit einem Methylzweig, eine optisch aktive Verbindung ist.

3. Biphenylverbindung nach Anspruch 1, dadurch gekennzeichnet, daß sie in Racematform vorliegt.

4. Biphenylverbindung nach einem der voranstehenden Ansprüche, dadurch gekennzeichnet, daß X ein Wasserstoffatom oder ein Fluoratom oder ein Cyanogruppe ist.

**5.** Biphenylverbindung nach einem der voranstehenden Ansprüche, dadurch gekennzeichnet, daß die Alkylgruppe mit dem Methylzweig 8 oder 9 Kohlenstoffatome enthält.

**6.** Biphenylverbindung nach einem der voranstehenden Anprüche, dadurch gekennzeichnet, daß die Alkylgruppe, die von der Alkylgruppe mit dem Methylzweig verschieden ist, eine lineare $C_6$-$C_{10}$-Alkylgruppe ist.

**7.** Flüssigkristallzusammensetzung mit mindestens zwei Komponenten, von denen eine eine Biphenylverbindung nach einem der voranstehenden Ansprüche ist.

**8.** Flüssigkristallzusammensetzung nach Anspruch 7, dadurch gekennzeichnet, daß sie eine chirale smektische Phase zeigt.

**9.** Flüssigkristallzusammensetzung nach Anspruch 7, dadurch gekennzeichnet, daß sie eine chirale nematische Phase zeigt.

## Revendications

**1.** Composés biphényliques de la formule:

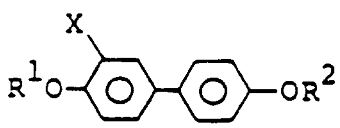

(I)

dans laquelle X représente un atome d'hydrogène, de fluor, de chlore ou de brome ou un groupement nitrile et, lorsque X est un atome de fluor, de chlore ou de brome ou un groupement nitrile, un des $R^1$ et $R^2$ représente un groupement alkyle en $C_4$ - $C_{18}$ ayant une seule chaîne latérale méthyle et l'autre des $R^1$ et $R^2$ représente un groupement alkyle en $C_1$ - $C_{20}$ ou, lorsque X représente un atome d'hydrogène, un des $R^1$ et $R^2$ représente un groupement alkyle ayant une chaîne latérale méthyle de la formule:

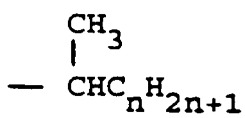

(dans laquelle n représente un nombre entier égal ou supérieur à 3) et l'autre des $R^1$ et $R^2$ représente un groupement alkyle en $C_1$ - $C_{20}$, en excluant le composé dans lequel $R^1$ est un groupement 6-méthyl-octyle, $R^2$ est un groupement n-décyle et X est un atome de fluor.

**2.** Composé biphénylique selon la revendication 1, caractérisé en ce qu'il est une substance optiquement active sur la base du groupement alkyle ayant une chaîne latérale méthyle.

**3.** Composé biphénylique selon la revendication 1, caractérisé en ce qu'il est dans la forme racémique.

**4.** Composé biphénylique selon l'une quelconque des revendications précédentes, caractérisé en ce que X est un atome d'hydrogène ou de fluor ou un groupement nitrile.

**5.** Composé biphénylique selon l'une quelconque des revendications précédentes, caractérisé en ce que le groupement alkyle ayant une chaîne latérale méthyle contient 8 ou 9 atomes de carbone.

**6.** Composé biphénylique selon l'une quelconque des revendications précédentes, caractérisé en ce que le groupement alkyle autre que le groupement alkyle ayant une chaîne latérale méthyle est un groupement alkyle linéaire en $C_6$ - $C_{10}$.

7. Composition de cristaux liquides comprenant au moins deux composants dont au moins un est un composé biphénylique tel que revendiqué dans l'une quelconque des revendications précédentes.

8. Composition de cristaux liquides selon la revendication 7, caractérisée en ce qu'elle présente une phase smectique chirale.

9. Composition de cristaux liquides selon la revendication 7, caractérisée en ce qu'elle présente une phase nématique chirale.